# FASCICULE DE BREVET EUROPEEN

(11) **EP 3 876 878 B1**
(45) Date de publication et mention de la délivrance du brevet: **05.10.2022**
(21) Numéro de dépôt: 19820824.1
(22) Date de dépôt: 05.11.2019
(51) Int. Cl.: A61F 5/01

(54) **ORTHESE SOUPLE POUR POIGNET**
BIEGSAM ORTHOSE FÜR DAS HANDGELENK
FLEXIBLE ORTHOSIS FOR WRIST

(30) Priorité: 07.11.2018 FR 1860254
(43) Date de publication de la demande: 15.09.2021
(73) Titulaire: Millet Innovation, 26270 Loriol sur Drome (FR)
(72) Inventeur: TREPIER-LE BELLER, Maria Luisa, 26800 PORTES-LES-VALENCE (FR)
(74) Mandataire: Marchand, André
(86) Numéro de dépôt international: PCT/FR2019/052615
(87) Numéro de publication internationale: WO 2020/094968

(56) Documents cités:
- EP-A1- 3 041 441
- EP-B1- 3 041 441
- WO-A1-97/12570
- US-A- 5 413 553

## Description

La présente invention concerne une orthèse pour une articulation et plus particulièrement l'articulation du poignet.

Depuis des dizaines d'années, l'orthopédie s'est consacrée à des pathologies nécessitant des moyens de contention et/ou des moyens d'immobilisation. Depuis quelques années, on cherche à "économiser" les articulations dans toutes leurs composantes, sans perturber leur usage habituel. C'est ainsi par exemple que pour l'arthrose du pouce, la Demanderesse a développé une orthèse légère minimisant les mouvements involontaires du pouce en appliquant des principes de mécanique (brevet EP 3 041 441).

Or, il est apparu qu'une telle orthèse légère pouvait être bénéfique non seulement pour soulager les douleurs liées à l'arthrose, mais également pour une pathologie dont l'occurrence est tout aussi importante que pour l'arthrose du pouce. Il s'agit du syndrome du canal carpien dont la grande fréquence tient à la multiplicité des facteurs de risques et à l'effet de leur combinatoire. Dans tous les cas, il s'agit de limiter les pressions sur le nerf médian dans le canal carpien à l'intérieur duquel passe notamment les tendons fléchisseurs de la main accompagnés de leur gaine synoviale. Le syndrome du canal carpien se caractérise par des sensations de fourmillement, de picotements, d'engourdissements ou de décharges électriques principalement localisées sur la face palmaire des trois premiers doigts (territoire anatomique du nerf médian) et par une perte de force musculaire dans le poignet et la main affectés. Ces gênes sont souvent ressenties la nuit, réveillant le patient. Elles peuvent également apparaitre dans la journée, déclenchées par certains mouvements ou le maintien de la main dans certaines positions néfastes.

Il s'avère que certains mouvements et postures favorisent l'apparition du syndrome du canal carpien, sa persistance et/ou son aggravation. Une solution simple pour soulager et limiter l'évolution, voire résoudre cette affection consiste à limiter la fréquence et l'amplitude de ces mouvements et postures à risque dans la vie courante. Certains gestes sont particulièrement contraignants pour le nerf médian en cause dans le syndrome du canal carpien. En particulier, les angulations extrêmes du poignet ont pour effet de comprimer le canal carpien de façon aiguë contre le ligament annulaire du carpe lors de la flexion (côté face palmaire de la main) et contre les os du carpe lors de l'extension (côté face dorsale de la main). Le nerf médian peut également subir une compression chronique résultant fréquemment d'une inflammation des tendons des muscles fléchisseurs des doigts et/ou de leur gaine synoviale et de l'augmentation de leur volume.

Le syndrome du canal carpien est généralement traité dans un premier temps par le port d'une attelle de poignet la nuit, et/ou par des infiltrations (injections de cortisone dans le canal carpien). Les attelles qui sont généralement utilisées, immobilisent l'articulation du poignet en position neutre, ce qui permet d'éviter des postures pouvant comprimer de façon particulièrement importante le nerf médian. Le principal inconvénient de ces attelles réside dans leur rigidité qui empêche un usage normal de la main et du poignet. Par conséquent, elles sont très inconfortables et il est néfaste pour le patient de porter une telle attelle en situation d'activité courante nécessitant un travail de la main. Des compensations sur d'autres articulations ainsi que des efforts anormalement élevés seraient alors nécessaires pour effectuer ces tâches courantes, ce qui peut provoquer l'apparition de pathologies secondaires.

Il est donc souhaitable de proposer une orthèse susceptible d'une part de minimiser l'amplitude des mouvements de l'articulation du poignet pendant les phases de position réflexe neutre, et d'autre part d'autoriser l'amplitude des mouvements de l'articulation dans les phases de mouvements volontaire liées aux activités courantes et ceci dans tous les axes de rotation possibles. Il peut être également souhaitable d'amortir les mouvements volontaires, notamment pour accélérer le retour à la position neutre.

Des modes de réalisation concernent un orthèse de main et de poignet comprenant : un manchon comportant une partie distale, et une partie proximale, la partie proximale étant conformée pour épouser la forme d'une partie de l'avant-bras s'étendant jusqu'au poignet, la partie distale étant conformée pour épouser la forme d'une partie de la main s'étendant depuis la base des articulations métacarpo-phalangiennes des doigts jusqu'au poignet, la partie distale comportant une première ouverture pour le passage du pouce, une seconde ouverture pour le passage des autres doigts, la partie proximale étant conformée pour épouser la forme du poignet et d'une partie de l'avant-bras couverte par le manchon, le manchon étant conformé pour contraindre le poignet à l'encontre d'un mouvement de flexion du côté de la face palmaire de la main, le manchon étant réalisé en un matériau élastique, et une bande de maintien en un matériau élastique, fixée sur les parties distale et proximale du manchon et adaptée à couvrir seulement une partie supérieure de la région la plus fine du poignet entre des parties latérales opposées du poignet.

Selon un mode de réalisation, le manchon est réalisé à partir d'une seule pièce comportant une partie dorsale prévue pour couvrir la partie dorsale de la main et du poignet, une partie palmaire prévue pour couvrir une partie palmaire de la main et du poignet, et une bande reliant les parties dorsale et palmaire.

Selon un mode de réalisation, le manchon comprend une fente formée dans la partie dorsale entre les parties proximale et distale du manchon, et s'étendant entre deux bords opposés de la partie dorsale, des bords opposés de la fente étant fixés entre eux afin de refermer la fente, la bande de maintien étant fixée sur la fente une fois refermée.

Selon un mode de réalisation, la fente est réalisée en forme de fuseau en retirant une partie du matériau formant le manchon, ou est réalisée sans retrait de matériau formant le manchon, les bords opposés de la fente étant fixés l'un sur l'autre afin de réaliser une zone de chevauchement en forme de fuseau du matériau formant le manchon.

Selon un mode de réalisation, le manchon est assemblé par deux lignes de fixation venant se positionner sur des régions latérales du poignet et de la main, chacune des lignes de fixation liant un bord de la partie dorsale avec un bord plus long de la partie palmaire.

Selon un mode de réalisation, les deux lignes de fixation comprennent une ligne de fixation prévue pour s'étendre le long d'une face latérale externe de la main en passant côté distal sur la face dorsale de la main.

Selon un mode de réalisation, la première ouverture du manchon pour le passage du pouce, présente une échancrure formée dans une partie dorsale du manchon dans le prolongement du pouce pour ne pas gêner des mouvements latéraux du pouce.

Selon un mode de réalisation, une partie dorsale du manchon comporte un pli débouchant dans la première ouverture et s'étendant dans le prolongement du pouce pour laisser libres des mouvements latéraux du poignet et d'ouverture de la main.

Selon un mode de réalisation, le manchon est réalisé en plusieurs tailles afin qu'un bord distal du manchon soit étiré transversalement d'au plus 20% et que la partie proximale du manchon soit étirée transversalement d'au plus 10%.

Selon un mode de réalisation, la pièce formant le manchon présente au moins l'une des caractéristiques suivantes : la pièce présente suivant l'axe longitudinal du manchon, un module d'Young compris entre 0,5 Mpa et 1 Mpa, la pièce présente une épaisseur comprise entre 1 mm et 1,4 mm.

Selon un mode de réalisation, la pièce formant le manchon comprend sur toute son étendue deux couches de tissu élastique assemblées par collage.

Selon un mode de réalisation, chacune des deux couches de tissu est réalisée dans un tissu présentant au moins l'une des caractéristiques suivantes : le tissu comprend entre 75 et 85% en poids de polyamide et entre 15 et 25% en poids d'élasthanne, le tissu présente une élasticité de 85 à 115% dans le sens de la chaine et de 65 à 95% dans le sens de la trame du tissu, la chaine du tissu étant orientée axialement sur la partie dorsale du manchon.

Selon un mode de réalisation, la bande de maintien est réalisée dans un matériau élastique similaire au matériau formant le manchon.

Selon un mode de réalisation, la bande de maintien présente au moins l'une des caractéristiques suivantes : la bande de maintien est liée au manchon par une couture en zigzag afin de ne pas empêcher une extension élastique de l'orthèse dans le sens de la longueur de la bande de maintien, la bande de maintien présente une largeur comprise entre 0,3 et 1,5 cm, la bande de maintien recouvre entre 30 et 70% de la circonférence du poignet, la bande de maintien est fixée au manchon uniquement à une extrémité et fixée à une autre extrémité par un dispositif de serrage pour ajuster la tension de la bande de maintien autour du poignet, la bande de maintien est réalisée en un tissu élastique enduit d'une couche en un gel polymère.

Selon un mode de réalisation, l'orthèse comprend un dispositif de serrage du manchon autour de l'avant-bras.

Des exemples de réalisation de l'invention seront décrits dans ce qui suit, à titre non limitatif en relation avec les figures jointes parmi lesquelles :
les figures 1A et 1B sont respectivement des vues de dessus et latérale d'une main portant une orthèse selon un mode de réalisation,
la figure 2 représente la forme d'une pièce de faible épaisseur qui est assemblée pour former l'orthèse de la figure 1A, selon un mode de réalisation,
la figure 3 représente des chronogrammes de mouvements du poignet, avec et sans l'orthèse,
la figure 4 représente des chronogrammes de la vitesse du mouvement du poignet, avec et sans l'orthèse.

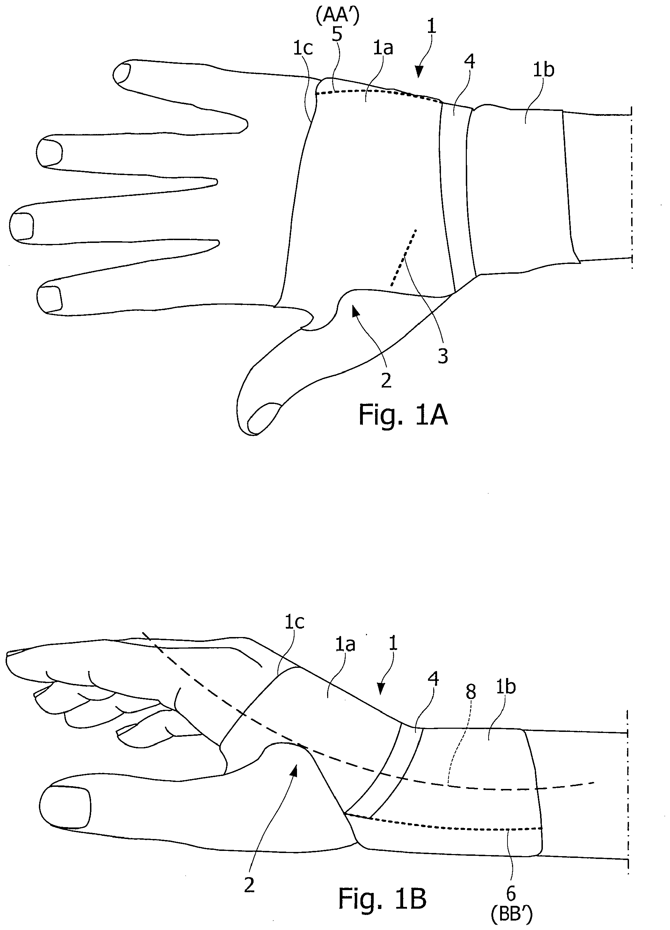

Les figures 1A et 1B représentent une orthèse selon un mode de réalisation. L'orthèse comprend un manchon 1 couvrant le poignet et une partie de l'avant-bras, et s'étendant jusqu'à la naissance des articulations des doigts. Le manchon comprend une partie distale 1a et une partie proximale 1b, et chacune de ces parties comporte une partie dorsale et une partie palmaire. La partie distale 1a du manchon est conformée pour épouser la forme de la partie de la main s'étendant depuis la base des articulations métacarpo-phalangiennes des doigts jusqu'à la limite du poignet. La partie distale 1a comporte un bord distal 1c formant une première ouverture pour le passage du pouce et une seconde ouverture pour le passage des autres doigts, à savoir l'index, le majeur, l'annulaire, et l'auriculaire. La partie proximale 1b est conformée pour épouser la forme du poignet et de la partie de l'avant-bras couverte par l'orthèse 1.

Une échancrure fonctionnelle 2 peut être réalisée dans la partie dorsale de la partie 1a du manchon 1, au niveau du pouce. L'échancrure 2 permet de laisser libre la gestuelle du pouce et les mouvements latéraux du poignet, notamment l'abduction.

Un pli fonctionnel 3 peut être volontairement positionné sur la partie dorsale du manchon 1, à partir de l'ouverture pour le passage du pouce, dans le prolongement de ce dernier, afin de ne pas gêner des mouvements latéraux du poignet et de l'ouverture de la main.

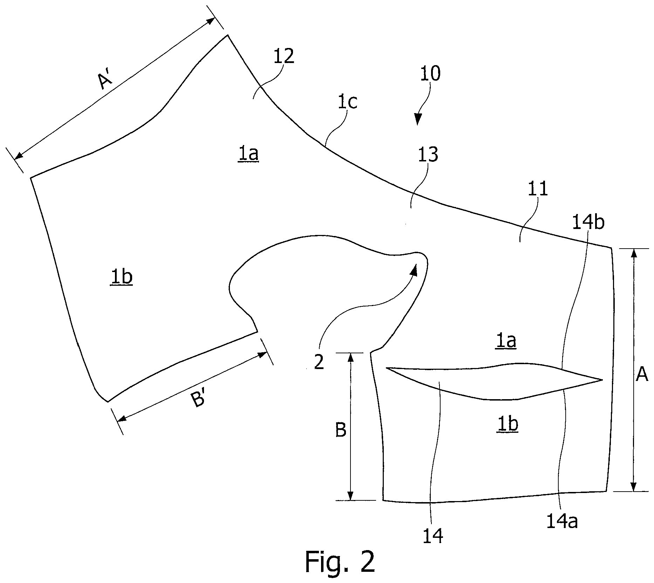

La figure 2 représente une pièce 10 en un matériau élastique de faible épaisseur, qui est assemblée pour former le manchon 1, selon un mode de réalisation. La pièce 10 comprend une partie dorsale 11, et une partie palmaire 12 reliées entre elles par une bande 13. Les parties dorsale 11 et palmaire 12 sont conformées pour couvrir et épouser respectivement la forme de la partie dorsale de la main, du poignet et d'une partie de l'avant-bras, et la forme de la partie palmaire de la main, du poignet et de l'avant-bras. La partie dorsale 11 de la pièce 10 comporte une fente 14 en forme de fuseau s'étendant entre des bords d'assemblage A et B de la partie dorsale 11. La fente 14 est réalisée dans la zone de la partie dorsale 11 destinée à couvrir le poignet.

La partie palmaire 12 de la pièce 10 comporte des bords d'assemblage A', B'. Les bords d'assemblage A et A' sont prévus pour être fixés ensemble et pour se placer le long d'une région latérale externe de la partie couverte par le manchon 1, de l'avant-bras du poignet et de la main. Les bords d'assemblage B et B' sont prévus pour être fixés ensemble et pour se placer le long d'une région latérale interne de la partie couverte par le manchon 1, de l'avant-bras, du poignet, jusqu'à la base du pouce.

La bande 13 de la pièce 10 est conformée pour couvrir la main entre les régions dorsale et palmaire de la main en passant par la région de pli entre le pouce et l'index. L'échancrure 2 est réalisée dans la bande 13.

Les bords 14a, 14b de la fente 14 sont assemblés bord à bord. De plus, les bords A et B de la pièce 10 sont plus courts que les bords A' et B'. Ces deux dispositions apportent une augmentation de l'ergonomie et du confort à l'orthèse 1. En effet, elles lui donnent un galbe 8 qui épouse la forme de la main en la contraignant à l'encontre du mouvement de flexion du côté de la face palmaire de la main.

La couture latérale externe assemblant les bords A, A' de l'orthèse 1 peut être volontairement décalée côté distal vers le dos de la main pour minimiser les efforts des mouvements latéraux du poignet, notamment l'abduction. La finition de cette couture est de préférence plate et non-agressive pour garantir le confort au porté.

La réalisation du manchon 1 à partir d'une seule pièce (10) permet d'éviter la présence d'une couture au voisinage de la région de pli entre le pouce et l'index, une telle couture formant inévitablement une rigidité pouvant gêner les mouvements du pouce et en particulier la fonction de pince de la main. Toutefois, il est possible de réaliser le manchon à partir de deux pièces et d'assembler ces deux parties notamment par une couture réalisée en un emplacement susceptible de ne pas provoquer de gêne, par exemple en coupant la pièce 10 dans le prolongement de l'échancrure 2, et/ou dans la partie dorsale 11.

Une bande de maintien 4 est positionnée en regard du poignet et fait le demi-tour de celui-ci du côté dorsal de la main. La bande de maintien 4 couvre ainsi la couture refermant la fente 14 et assure le bon positionnement de l'orthèse entre la main et l'avant-bras. Elle optimise également l'accompagnement progressif du poignet en flexion sans imposer de pression importante sur la zone du canal carpien et tout en permettant une extension du poignet avec le minimum de contraintes.

En effet, l'ajout de la bande de maintien 4 augmente la rigidité de l'orthèse dans une zone très précise. Lors du mouvement de flexion du poignet du côté de la face palmaire de la main, la circonférence de celui-ci augmente. Or cette augmentation de la circonférence du poignet n'est pas répartie également sur tout le pourtour du poignet. En effet, l'arc décrit par la face dorsale du poignet augmente de longueur alors que l'arc décrit par la face palmaire du poignet diminue en longueur. Ainsi, la bande de maintien 4 est positionnée le long de l'arc de la face dorsale du poignet le long de la région du poignet la plus fine, c'est-à-dire juste au-dessus de la protubérance styloïde du côté avant-bras et juste en dessous de l'articulation carpo-métacarpienne du pouce du côté main. La bande de maintien 4 induit donc une augmentation de la résistance de l'orthèse 1 face à la déformation du poignet le long de la bande 4 et donc une augmentation de la tension sur la bande 4 qui vient à l'encontre du mouvement de flexion. En revanche, rien n'est ajouté sur la face palmaire du poignet, qui ne comprend donc qu'une simple épaisseur du matériau dans lequel la pièce 10 est formée, afin que l'orthèse puisse absorber les déformations sans imposer d'augmentation de pression importante en regard de la zone du canal carpien.

Par ailleurs, comme la bande de maintien 4 se trouve positionnée autour de la région la plus étroite du poignet, l'orthèse se trouve idéalement positionnée et ne peut que difficilement se déplacer axialement en direction de la main ou de l'avant-bras sans aide extérieure. Ainsi, la bande de maintien 4 crée un premier point fixe sous l'articulation carpo-métacarpienne et rigidifie l'orthèse sur la partie supérieure de la région la plus fine du poignet. Le positionnement de l'orthèse est également borné par la bande 13 passant sur la zone de pli entre le pouce et l'index, empêchant l'orthèse de se déplacer en direction de l'avant-bras, et donc constituant un second point fixe. Or, la distance entre la zone de pli du pouce et la zone la plus étroite du poignet augmente avec la flexion du poignet. La bande de maintien 4 impose donc que l'allongement longitudinal de la face dorsale du manchon 1 accompagnant le mouvement de flexion du poignet du côté de la face palmaire de la main, soit réalisée entre ces deux points fixes sur la partie dorsale. La longueur de l'orthèse sur laquelle la déformation se réalise est donc limitée, ce qui augmente la résistance de l'orthèse face au mouvement de flexion du poignet. Ces deux phénomènes viennent s'ajouter à l'effet poutre de l'orthèse recouvrant les membres de part et d'autre de l'articulation pour générer une résistance modérée mais significative face à la flexion du poignet, on obtient ainsi une limitation de l'amplitude ainsi que de la vitesse des mouvements de flexion du poignet non volontaires et une sensation de maintien lors des mouvements volontaires sans pour autant les entraver. Cette limitation de l'entrave au mouvement de flexion est réalisée en contraignant le moins possible ce mouvement dans la plage des angles de confort. D'après la norme sur l'ergonomie et plus particulièrement la manutention manuelle ISO 11228-3, l'angle de confort du poignet en flexion ne dépasse pas 45° depuis une position neutre.

La résistance que l'orthèse oppose au mouvement d'extension du poignet (du côté de la face dorsale de la main) est minimisée par sa configuration. En effet, l'effet poutre est toujours présent en résistance face à ce mouvement d'extension, mais la face palmaire de l'orthèse ne présente pas de rigidification localisée (pas de bande de maintien). Ainsi, la déformation induite par l'extension du poignet est absorbée sur une longueur (dans la direction axiale du manchon 1) beaucoup plus importante. De plus, la bande de maintien 4 n'empêche pas le tissu de faire des plis sur la face dorsale de l'orthèse pour absorber la diminution de longueur, en raison de sa faible largeur et de son positionnement le long du contour du poignet et sur la face dorsale du manchon 1 uniquement.

Ainsi, la bande de maintien 4 exerce une résistance significative face à un mouvement de flexion du poignet (du côté de la face palmaire de la main), sans augmenter la résistance face à un mouvement d'extension du poignet (du côté de la face dorsale de la main), ce dernier mouvement étant extrêmement sensible face à la contrainte. La pression exercée par l'orthèse en regard de la zone du canal carpien se trouve également minimisée.

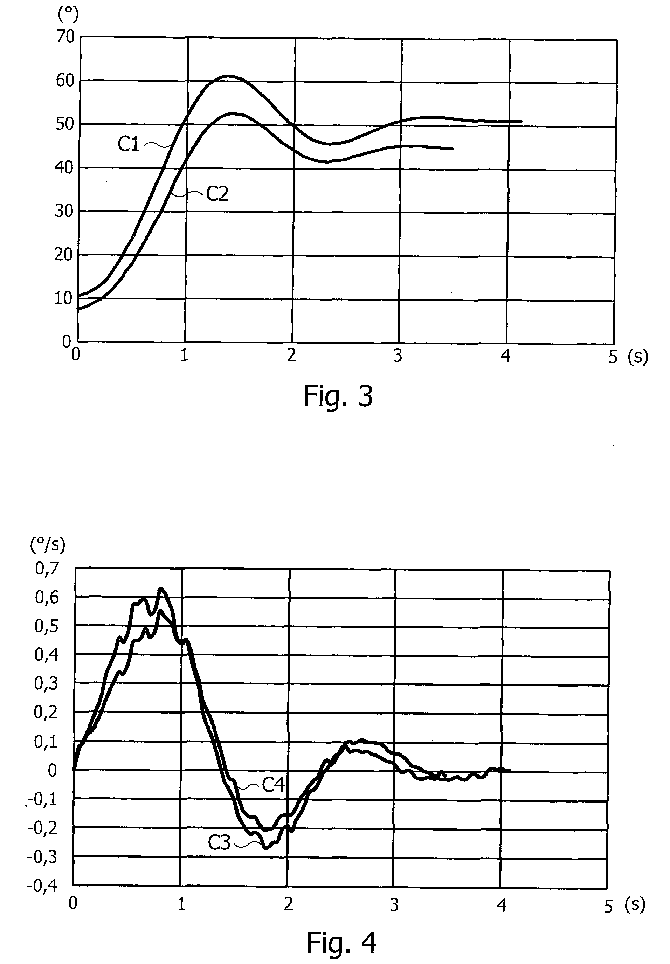

La figure 3 représente des chronogrammes C1, C2 de mouvements de flexion non contrôlés de la main sous l'effet de la gravité, la main étant placée au départ sensiblement horizontalement, l'avant-bras étant maintenu fixe dans le prolongement naturel de la main. L'amplitude du mouvement de flexion de la main est mesurée par l'angle de flexion. Les courbes C1, C2 ont été obtenues respectivement en l'absence et en présence de l'orthèse.

Les courbes C1, C2 présentent chacune un premier pic et un premier creux. En comparant les angles de flexion correspondant à ces premiers pics et premiers creux, il apparaît que le port de l'orthèse (courbe C2) entraine une diminution de l'angle de flexion du premier pic d'environ 10,1° (soit 16.5%). L'angle de flexion au premier creux est également diminué d'environ 5,6° (soit 12.2%). On peut également observer une diminution de l'écart entre l'amplitude maximum et l'amplitude minimum d'environ 29,2% et une diminution de l'angle de flexion une fois le mouvement terminé d'environ 7,7° (soit 15%), la position finale étant atteinte plus rapidement avec l'orthèse. En outre, les mouvements du poignet semblent se stabiliser plus rapidement avec le port de l'orthèse. En effet, l'angle de flexion se stabilise à environ 3,4 s avec l'orthèse et vers 3,8 s sans orthèse.

Il est à noter que les points de départ des courbes C1, C2 sont différents, le point de départ de la courbe C2 étant situé à environ 3° au-dessus de celui de la courbe C1. Cela signifie que la main part d'une position initiale dans le cas de la courbe C2 plus élevée que dans le cas de la courbe C1, ce qui aurait normalement tendance à augmenter au moins la valeur du premier pic de la courbe C2 et donc à minimiser les écarts de position angulaire entre les courbes C1 et C2. Cette différence de position initiale montre également l'effet de précontrainte exercée par l'orthèse à l'encontre des mouvements de flexion du poignet, qui a ainsi tendance à redresser la main.

La figure 4 représente des chronogrammes C3, C4 de variation de la vitesse angulaire de mouvements de flexion non contrôlés de la main sous l'effet de la gravité, la main étant placée au départ sensiblement horizontalement, l'avant-bras étant maintenu fixe dans le prolongement naturel de la main. Les courbes C3, C4 ont été obtenues respectivement en l'absence et en présence de l'orthèse. Les courbes C3, C4 présentent un premier pic de vitesse correspondant à la chute de la main, puis un passage par un point à une vitesse nulle correspondant à la position du poignet en flexion maximale après la chute. Ensuite, apparaît un premier creux correspondant au rebond de la main à l'inverse de la chute. Les pics et creux qui suivent correspondent aux oscillations suivantes de moindre amplitude et s'amortissant. En comparant les courbes C3 (sans orthèse) et C4 (avec orthèse), les vitesses correspondant au premier pic et au premier creux sont réduites grâce à l'orthèse, respectivement d'environ 13% pour le premier pic et d'environ 26% pour le premier creux. On remarque également que l'écart entre le premier creux et le premier pic est significativement réduit d'environ 17%, grâce à l'orthèse. Il peut être observé également que les temps entre ces pics et ces creux sont sensiblement les mêmes avec et sans orthèse (environ 1 s), ce qui implique que l'orthèse réduit également l'accélération angulaire d'une manière significative.

L'analyse de ces mesures montre également que le port de l'orthèse permet de diminuer les valeurs extrêmes des amplitudes, vitesses et accélérations des mouvements passifs et inconscients du poignet, et ce sans gêner les mouvements volontaires.

Selon un mode de réalisation, le manchon 1 est conformé pour exercer une compression limitée sur les tissus sous-jacents de la main, afin de ne pas provoquer de pathologies secondaires ou d'inconfort. Ce résultat est atteint en prévoyant plusieurs tailles du manchon afin de limiter l'étirement transversal du bord distal 1a du manchon compris entre 0 et 20% et de préférence entre 2,5 et 17,5%. Le choix de la taille étant effectué en mesurant la circonférence de la main dans la région couverte par la partie distale 1a du manchon. Par ailleurs, la circonférence du manchon 1 autour de la région la plus fine du poignet a été dimensionnée pour les différentes tailles, pour que l'allongement transversal de la partie proximale 1b du manchon reste inférieur à 10%, afin que celle-ci soit la moins compressive tout en restant au contact de la peau.

Selon un mode de réalisation, la pièce 10 formant le manchon 1 est réalisée dans un matériau élastique présentant, suivant l'axe longitudinal du manchon, un module d'Young compris entre 0,5 Mpa et 1 Mpa.

Selon un mode de réalisation, la pièce 10 formant le manchon 1 comprend sur toute son étendue deux couches de tissu élastique assemblées par collage.

Selon un mode de réalisation, chacune des deux couches formant la pièce 10 est réalisée dans un tissu comprenant entre 75 et 85% en poids de polyamide et entre 15 et 25% en poids d'élasthanne.

Selon un mode de réalisation, chacune des deux couches formant la pièce 10 présente un grammage de 155 g/m², et/ou une épaisseur comprise entre 0,5 et 0,7 mm, l'une ou l'autre de ces dispositions permettant d'apporter à l'usager une présence sécurisante de l'orthèse.

Selon un mode de réalisation, chacune des deux couches de tissu présente une élasticité de 85 à 115% dans le sens de la chaine, et de 65 à 95% dans le sens de la trame du tissu, la chaine du tissu étant orientée axialement sur la partie dorsale du manchon.

Selon un mode de réalisation, le manchon 1 est réalisé en un matériau présentant une épaisseur comprise entre 1 mm et 1,4 mm.

Selon un mode de réalisation, la bande de maintien 4 est réalisée en un matériau élastique ayant des propriétés mécaniques similaires à celles du matériau utilisé pour la réalisation du manchon 1.

Selon un mode de réalisation, la bande de maintien 4 est liée au manchon 1 par une couture en zigzag afin de ne pas empêcher une extension élastique de l'orthèse dans le sens de la longueur de la bande de maintien 4.

Selon un mode de réalisation, la bande de maintien 4 présente une largeur comprise entre 0,3 et 1,5 cm.

Selon un mode de réalisation, la bande de maintien 4 recouvre entre 30 et 70% de la circonférence du poignet.

Il apparaîtra clairement à l'homme de l'art que la présente invention est susceptible de diverses variantes de réalisation et diverses applications.

En particulier, l'invention n'est pas limitée à une orthèse visant à soulager le syndrome du canal carpien, mais elle peut s'appliquer à toute pathologie dans laquelle seule une précontrainte de la position du poignet dans sa position neutre est suffisante.

Par ailleurs, d'autres dispositions que la formation de la fente 14, et/ou la prévision de bords (A-A', B-B') de différentes longueurs pour les bords assemblés de la pièce 10 peuvent aisément être imaginés pour réaliser la précontrainte exercée par l'orthèse sur l'articulation du poignet pour maintenir la main dans sa position neutre par rapport à l'avant-bras. Par exemple, il peut être prévu de fixer un ou plusieurs renforts longitudinaux en matériau élastique le long de l'axe longitudinal de l'orthèse. Cependant, ces renforts ne doivent pas entraver les mouvements volontaires du poignet et de la main, tant que ces mouvements ne sont pas excessifs.

La bande de maintien 4 peut être réalisée en un matériau élastique enduit d'un matériau en un gel polymère tel qu'un gel de silicone. Elle peut également comprendre un ou plusieurs embranchements s'étendant vers la partie distale et/ou vers la partie proximale.

La bande de maintien 4 peut être fixée au manchon 1 seulement à une extrémité et fixée de manière réglable à son autre extrémité par exemple par dispositif à crochets et à boucles (Velcro^{®}) afin de permettre un réglage de sa tension.

Au lieu de fermer l'ouverture 14 en fixant l'un contre l'autre les bords 14a, 14b de l'ouverture, il peut être prévu de fermer l'ouverture 14 au moyen d'une pièce en un autre matériau élastique.

L'ouverture 14 peut avoir été réalisée sans retrait de tissu de sorte qu'elle se présente sous la forme d'une fente à bords parallèles (lorsque le tissu est disposé à plat sans subir d'étirement), l'ouverture étant refermée en tirant l'un deux les bords 14a, 14b en direction de l'autre bord afin d'obtenir un chevauchement de parties du manchon le long de l'ouverture 14. Une bande peut recouvrir ce chevauchement.

Il peut également être prévu un moyen de serrage, par exemple une sangle solidaire du manchon associée à un dispositif de fixation, par exemple à crochets et à boucles, pour serrer la partie proximale 1b du manchon 1 autour de la partie de l'avant-bras recouverte par l'orthèse.

L'échancrure 2 formée dans le bord dorsal de l'ouverture du manchon pour le passage du pouce, peut être omise, sachant que l'absence de cette disposition n'entraine pas de gêne particulière. Il en est de même du pli 3.

## Revendications

1. Orthèse de main et de poignet comprenant :
un manchon (1) comportant une partie distale (1a), et une partie proximale (1b), la partie proximale étant conformée pour épouser la forme d'une partie de l'avant-bras s'étendant jusqu'au poignet, la partie distale étant conformée pour épouser la forme d'une partie de la main s'étendant depuis la base des articulations métacarpo-phalangiennes des doigts jusqu'au poignet, la partie distale comportant une première ouverture pour le passage du pouce, une seconde ouverture pour le passage des autres doigts, la partie proximale étant conformée pour épouser la forme du poignet et d'une partie de l'avant-bras couverte par le manchon, le manchon étant conformé pour contraindre le poignet à l'encontre d'un mouvement de flexion du côté de la face palmaire de la main, le manchon étant réalisé en un matériau élastique, **caractérisé en ce qu'**il comprend en outre
une bande de maintien (4) en un matériau élastique, fixée sur les parties distale et proximale du manchon et adaptée à couvrir seulement une partie supérieure de la région la plus fine du poignet entre des parties latérales opposées du poignet.

2. Orthèse selon la revendication 1, dans laquelle le manchon (1) est réalisé à partir d'une seule pièce (10) comportant une partie dorsale (11) prévue pour couvrir la partie dorsale de la main et du poignet, une partie palmaire (12) prévue pour couvrir une partie palmaire de la main et du poignet, et une bande (13) reliant les parties dorsale et palmaire.

3. Orthèse selon la revendication 1 ou 2, dans laquelle le manchon (1) comprend une fente (14) formée dans la partie dorsale entre les parties proximale et distale du manchon, et s'étendant entre deux bords opposés de la partie dorsale, des bords opposés (14a, 14b) de la fente étant fixés entre eux afin de refermer la fente, la bande de maintien (4) étant fixée sur la fente une fois refermée.

4. Orthèse selon la revendication 3, dans laquelle la fente (14) est réalisée en forme de fuseau en retirant une partie du matériau formant le manchon, ou est réalisée sans retrait de matériau formant le manchon (1), les bords opposés (14a, 14b) de la fente étant fixés l'un sur l'autre afin de réaliser une zone de chevauchement en forme de fuseau du matériau formant le manchon.

5. Orthèse selon l'une des revendications 1 à 4, dans laquelle le manchon (1) est assemblé par deux lignes de fixation (5, 6) venant se positionner sur des régions latérales du poignet et de la main, chacune des lignes de fixation liant un bord (A, B) de la partie dorsale avec un bord (A', B') plus long de la partie palmaire.

6. Orthèse selon la revendication 5, dans laquelle les deux lignes de fixation (5, 6) comprennent une ligne de fixation (5) prévue pour s'étendre le long d'une face latérale externe de la main en passant côté distal sur la face dorsale de la main.

7. Orthèse selon l'une des revendications 1 à 6, dans laquelle la première ouverture du manchon (1) pour le passage du pouce, présente une échancrure (2) formée dans une partie dorsale du manchon dans le prolongement du pouce pour ne pas gêner des mouvements latéraux du pouce.

8. Orthèse selon l'une des revendications 1 à 7, dans laquelle une partie dorsale du manchon (1) comporte un pli (3) débouchant dans la première ouverture et s'étendant dans le prolongement du pouce pour laisser libres des mouvements latéraux du poignet et d'ouverture de la main.

9. Orthèse selon l'une des revendications 1 à 8, dans laquelle le manchon (1) est réalisé en plusieurs tailles afin qu'un bord distal du manchon soit étiré transversalement d'au plus 20% et que la partie proximale (1b) du manchon soit étirée transversalement d'au plus 10%.

10. Orthèse selon l'une des revendications 1 à 9, dans laquelle la pièce (10) formant le manchon (1) présente au moins l'une des caractéristiques suivantes :
la pièce présente suivant l'axe longitudinal du manchon, un module d'Young compris entre 0,5 Mpa et 1 Mpa, et
la pièce présente une épaisseur comprise entre 1 mm et 1,4 mm.

11. Orthèse selon l'une des revendications 1 à 10, dans laquelle la pièce (10) formant le manchon (1) comprend sur toute son étendue deux couches de tissu élastique assemblées par collage.

12. Orthèse selon la revendication 11, dans laquelle chacune des deux couches de tissu est réalisée dans un tissu présentant au moins l'une des caractéristiques suivantes :
le tissu comprend entre 75 et 85% en poids de polyamide et entre 15 et 25% en poids d'élasthanne, et
le tissu présente une élasticité de 85 à 115% dans le sens de la chaine et de 65 à 95% dans le sens de la trame du tissu, la chaine du tissu étant orientée axialement sur la partie dorsale du manchon.

13. Orthèse selon l'une des revendications 1 à 12, dans laquelle la bande de maintien (4) est réalisée dans un matériau élastique similaire au matériau formant le manchon (1).

14. Orthèse selon la revendication 13, dans laquelle la bande de maintien (4) présente au moins l'une des caractéristiques suivantes :
la bande de maintien est liée au manchon (1) par une couture en zigzag afin de ne pas empêcher une extension élastique de l'orthèse dans le sens de la longueur de la bande de maintien,
la bande de maintien présente une largeur comprise entre 0,3 et 1,5 cm,
la bande de maintien recouvre entre 30 et 70% de la circonférence du poignet,
la bande de maintien est fixée au manchon uniquement à une extrémité et fixée à une autre extrémité par un dispositif de serrage pour ajuster la tension de la bande de maintien autour du poignet, et
la bande de maintien est réalisée en un tissu élastique enduit d'une couche en un gel polymère.

15. Orthèse selon la revendication 14, comprenant un dispositif de serrage du manchon (1) autour de l'avant-bras.

## Patentansprüche

1. Hand- und Handgelenkorthese, umfassend:
eine Hülse (1), die einen distalen Teil (1a) und einen proximalen Teil (1b) aufweist, wobei der proximale Teil zum sich Anschmiegen an die Form eines Teils des Unterarms angepasst ist, der sich bis zu dem Handgelenk erstreckt, wobei der distale Teil zum sich Anschmiegen an die Form eines Teils der Hand angepasst ist, der sich von der Basis der Metacarpophalangealgelenke der Finger bis zu dem Handgelenk erstreckt, wobei der distale Teil eine erste Öffnung für den Durchgang des Daumens, eine zweite Öffnung für den Durchgang der anderen Finger aufweist, wobei der proximale Teil zum sich Anschmiegen an die Form des Handgelenks und eines Teils des Unterarms angepasst ist, der durch die Hülse bedeckt ist, wobei die Hülse zum Zwingen des Handgelenks gegen eine Beugungsbewegung aufseiten der Hohlhandfläche der Hand angepasst ist, wobei die Hülse aus einem elastischen Material hergestellt ist, **dadurch gekennzeichnet, dass** sie ferner
ein Halteband (4) aus einem elastischen Material umfasst, das an dem distalen und dem proximalen Teil der Hülse befestigt und geeignet ist, um nur einen Teil über der dünnsten Region des Handgelenks zwischen gegenüberliegenden lateralen Teilen des Handgelenks zu bedecken.

2. Orthese nach Anspruch 1, wobei die Hülse (1) aus einem einzigen Stück (10) hergestellt ist, das einen Rückenteil (11), der zum Bedecken des Rückenteils der Hand und des Handgelenks vorgesehen ist, einen Hohlhandteil (12), der zum Bedecken eines Hohlhandteils der Hand und des Handgelenks vorgesehen ist, und ein Band (13) aufweist, das den Rücken- und den Hohlhandteil verbindet.

3. Orthese nach Anspruch 1 oder 2, wobei die Hülse (1) einen Spalt (14) umfasst, der in dem Rückenteil zwischen dem proximalen und dem distalen Teil der Hülse ausgebildet ist, und sich zwischen zwei gegenüberliegenden Rändern des Rückenteils erstreckt, wobei die gegenüberliegenden Ränder (14a, 14b) des Spaltes miteinander befestigt werden, um den Spalt wieder zu schließen, wobei das Halteband (4) an dem Spalt befestigt wird, sobald er wieder geschlossen ist.

4. Orthese nach Anspruch 3, wobei der Spalt (14) spindelförmig hergestellt ist, indem ein Teil des Materials entfernt wird, das die Hülse ausbildet, oder ohne Entfernen von Material hergestellt ist, das die Hülse (1) ausbildet, wobei die gegenüberliegenden Ränder (14a, 14b) des Spaltes aneinander befestigt werden, um einen spindelförmigen Überlappungsbereich des Materials herzustellen, das die Hülse ausbildet.

5. Orthese nach einem der Ansprüche 1 bis 4, wobei die Hülse (1) durch zwei Befestigungslinien (5, 6) zusammengefügt ist, die sich an den lateralen Regionen des Handgelenks und der Hand positionieren, wobei jede der Befestigungslinien einen Rand (A, B) des Rückenteils mit einem längeren Rand (A', B') des Hohlhandteils verknüpft.

6. Orthese nach Anspruch 5, wobei die zwei Befestigungslinien (5, 6) eine Befestigungslinie (5) umfassen, die zum sich Erstrecken entlang einer äußeren lateralen Fläche der Hand im Vorbeigehen an der distalen Seite an der Rückenfläche der Hand vorgesehen ist.

7. Orthese nach einem der Ansprüche 1 bis 6, wobei die erste Öffnung der Hülse (1) für den Durchgang des Daumens eine Aussparung (2) vorweist, die in einem Rückenteil der Hülse in der Verlängerung des Daumens zum nicht Behindern von lateralen Bewegungen des Daumens ausgebildet ist.

8. Orthese nach einem der Ansprüche 1 bis 7, wobei ein Rückenteil der Hülse (1) eine Falte (3) aufweist, die in der ersten Öffnung mündet und sich in der Verlängerung des Daumens zum Freigeben von lateralen Bewegungen des Handgelenks und der Öffnung der Hand erstreckt.

9. Orthese nach einem der Ansprüche 1 bis 8, wobei die Hülse (1) in einer Vielzahl von Größen hergestellt ist, damit ein distaler Rand der Hülse um höchstens 20 % quer gestreckt wird und der proximale Teil (1b) der Hülse um höchstens 10 % quer gestreckt wird.

10. Orthese nach einem der Ansprüche 1 bis 9, wobei das Stück (10), das die Hülse (1) ausbildet, mindestens eines der folgenden Merkmale vorweist:
das Stück weist der Längsachse der Hülse folgend einen Elastizitätsmodul zwischen 0,5 Mpa und 1 Mpa vor, und
das Stück weist eine Dicke zwischen 1 mm und 1,4 mm vor.

11. Orthese nach einem der Ansprüche 1 bis 10, wobei das Stück (10), das die Hülse (1) ausbildet, an ihrer gesamten Ausdehnung zwei Schichten von elastischem Gewebe aufweist, die durch Kleben zusammengefügt sind.

12. Orthese nach Anspruch 11, wobei jede der zwei Gewebeschichten aus einem Gewebe hergestellt ist, das mindestens eines der folgenden Merkmale vorweist:
das Gewebe umfasst zu zwischen 75 und 85 Gew.-% Polyamid und zu zwischen 15 und 25 Gew.-% Elastan, und
das Gewebe weist eine Elastizität von 85 bis 115 % in der Kettrichtung und von 65 bis 95 % in der Schussrichtung des Gewebes vor, wobei die Kettfäden des Gewebes an dem Rückenteil der Hülse axial ausgerichtet sind.

13. Orthese nach einem der Ansprüche 1 bis 12, wobei das Halteband (4) aus einem elastischen Material hergestellt ist, das dem Material ähnelt, das die Hülse (1) ausbildet.

14. Orthese nach Anspruch 13, wobei das Halteband (4) mindestens eines der folgenden Merkmale vorweist:
das Halteband ist durch eine Zickzacknaht mit der Hülse (1) verknüpft, um eine elastische Dehnung der Orthese in der Richtung der Länge des Haltebandes nicht zu verhindern,
das Halteband weist eine Breite zwischen 0,3 und 1,5 cm vor,
das Halteband verdeckt zwischen 30 und 70 % des Umfangs des Handgelenks,
das Halteband ist einzig an einem Ende an der Hülse befestigt und an einem anderen Ende durch eine Festziehvorrichtung zum Einstellen der Spannung des Haltebands um das Handgelenk herum befestigt, und
das Halteband ist aus einem elastischen Gewebe hergestellt, das mit einer Schicht Polymergel beschichtet ist.

15. Orthese nach Anspruch 14, umfassend eine Vorrichtung zum Festziehen der Hülse (1) um den Unterarm herum.

## Claims

1. Hand and wrist orthosis comprising:
a sleeve (1) having a distal portion (1a) and a proximal portion (1b), the proximal portion being shaped to match the shape of a part of the forearm extending to the wrist, the distal portion being shaped to match the shape of a part of the hand extending from the base of the metacarpophalangeal joints of the fingers to the wrist, the distal portion having a first opening for the passage of the thumb, a second opening for the passage of the other fingers, the proximal portion being shaped to conform to the shape of the wrist and a portion of the forearm covered by the sleeve, the sleeve being shaped to constrain the wrist against a bending movement toward the palmar side of the hand, the sleeve being made of an elastic material; and further comprising
a support band (4) made of an elastic material, attached to the distal and proximal portions of the sleeve and adapted to cover only an upper part of the narrower region of the wrist between opposite lateral sides of the wrist.

2. The orthosis according to claim 1, wherein the sleeve (1) is made from a single piece (10) including a dorsal portion (11) adapted to cover the dorsal portion of the hand and wrist, a palmar portion (12) adapted to cover a palmar portion of the hand and wrist, and a band (13) connecting the dorsal and palmar portions.

3. The orthosis according to claim 1 or 2, wherein the sleeve (1) has a slit (14) formed in the dorsal portion between the proximal and distal portions of the sleeve, and extending between two opposite edges of the dorsal portion, the opposite edges (14a, 14b) of the slit being attached to each other to close the slit, the support band (4) being attached to the slit when closed.

4. The orthosis of claim 3, wherein the slit (14) is spindle-shaped by removal of a portion of the material forming the sleeve, or is formed without removal of material forming the sleeve (1), wherein the opposing edges (14a, 14b) of the slit are secured to each other to provide a spindle-shaped overlap area of the material forming the sleeve.

5. The orthosis according to any of claims 1 to 4, wherein the sleeve (1) is assembled by two attachment lines (5, 6) that are positioned on lateral regions of the wrist and hand, each of the attachment lines connecting an edge (A, B) of the dorsal part with a longer edge (A', B') of the palmar part.

6. The orthosis of claim 5, wherein the two attachment lines (5, 6) comprise an attachment line (5) adapted to extend along an outer lateral side of the hand passing distally over the dorsal side of the hand.

7. The orthosis according to one of claims 1 to 6, wherein the first opening of the sleeve (1) for the passage of the thumb has a notch (2) formed in a dorsal part of the sleeve in the prolongation of the thumb so as not to interfere with lateral movements of the thumb.

8. The orthosis according to one of claims 1 to 7, wherein a dorsal part of the sleeve (1) comprises a fold (3) extending from the first opening in the prolongation of the thumb to favor free lateral movements of the wrist and opening of the hand.

9. The orthosis according to any one of claims 1 to 8, wherein the sleeve (1) is made in a plurality of sizes so that a distal edge of the sleeve is stretched transversely by at most 20% and the proximal portion (1b) of the sleeve is stretched transversely by at most 10%.

10. The orthosis according to any of claims 1 to 9, wherein the material (10) forming the sleeve (1) has at least one of the following features:
the material has a Young's modulus along the longitudinal axis of the sleeve between 0.5 MPa and 1 MPa, and
the material has a thickness between 1 mm and 1.4 mm.

11. The orthosis according to one of claims 1 to 10, wherein the material (10) forming the sleeve (1) comprises, over its entire extent, two layers of elastic fabric assembled by gluing.

12. The orthosis of claim 11, wherein each of the two fabric layers is a fabric having at least one of the following features:
the fabric comprises between 75 and 85% by weight of polyamide and between 15 and 25% by weight of elastane, and
the fabric has an elasticity of 85 to 115% in the warp direction and 65 to 95% in the weft direction, wherein the warp of the fabric is oriented axially on the dorsal portion of the sleeve.

13. The orthosis according to any one of claims 1 to 12, wherein the support band (4) is made of an elastic material similar to the material forming the sleeve (1).

14. The orthosis of claim 13, wherein the support band (4) has at least one of the following features:
the support band is connected to the sleeve (1) by a zigzag seam so as not to interfere with elastic extension of the orthosis in the longitudinal direction of the support band,
the support band has a width between 0.3 and 1.5 cm,
the support band covers between 30 and 70% of the circumference of the wrist,
the support band is attached to the sleeve only at one end and secured at another end by a tightening device to adjust the tension of the support band around the wrist, and
the support band is made of an elastic fabric coated with a layer of a polymer gel.

15. The orthosis of claim 14, comprising a device for tightening the sleeve (1) around the forearm.
